(19) Europäisches Patentamt
European Patent Office
Office européen des brevets

(11) Publication number: **0 054 330**
**B1**

# (12) EUROPEAN PATENT SPECIFICATION

(45) Date of publication of patent specification: **17.09.86**

(51) Int. Cl.[4]: **C 12 N 15/00,** C 12 P 21/02, C 12 N 1/20, C 07 H 21/04

(21) Application number: **81201354.8**

(22) Date of filing: **11.12.81**

## (54) DNA sequences encoding various allelic forms of mature thaumatin, recombinant plasmids comprising said DNA's and a process for their preparation, bacterial cultures comprising said recombinant plasmids, and method for producing mature thaumatin.

(30) Priority: **12.12.80 GB 8039855**

(43) Date of publication of application:
**23.06.82 Bulletin 82/25**

(45) Publication of the grant of the patent:
**17.09.86 Bulletin 86/38**

(84) Designated Contracting States:
**AT BE CH DE FR GB IT LI NL SE**

(56) References cited:
**EP-A-0 040 466**
**GB-A-1 565 190**
**US-A-4 237 224**

**NATURE, vol. 284, no. 5758, April 24, 1980 "Gene machining sweeter gum" page 653**

**NATURE, vol. 281, October 18, 1979 D.V. GOEDDEL et al.: "Direct expression in Escherichia coli of a DNA sequence coding for human growth hormone" page 544**

**CHEMICAL ABSTRACTS, vol. 91, no. 13, September 24, 1979 abstract 118895c, page 190**

**COLUMBUS, OHIO (US) IYENGAR RAMANUJA B. et al.: "The complete amino acid sequence of the sweet protein thaumatin.I."**

(73) Proprietor: **UNILEVER NV**
**Burgemeester s'Jacobplein 1 P.O. Box 760**
**NL-3000 DK Rotterdam (NL)**
(84) **BE CH DE FR IT LI NL SE AT**

(73) Proprietor: **UNILEVER PLC**
**Unilever House Blackfriars P.O. Box 68**
**London EC4P 4BQ (GB)**
(84) **GB**

(72) Inventor: **Verrips, Cornelis Theodorus**
**Hagedoorn 18**
**NL-3142 KB Maassluis (NL)**
Inventor: **Ledeboer, Adrianus Marinus**
**Burg. le Fèvre de Montignyplein 8**
**NL-3055 NL Rotterdam (NL)**
Inventor: **Edens, Luppo**
**Albert Schweitzerdreef 29**
**NL-3146 AA Maassluis (NL)**
Inventor: **Klok, Robert**
**Wilgendreef 29**
**NL-3137 CZ Vlaardingen (NL)**
Inventor: **Maat, Jan**
**Molenstraat 2**
**NL-2681 BS Monster (NL)**

(74) Representative: **van der Toorren, Johannes, Drs. et al**
**UNILEVER N.V. Patent Division Postbus 137**
**NL-3130 AC Vlaardingen (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 054 330 B1

(56) References cited:

**NUCLEIC ACIDS SYNTHESIS- Symposium Series, no. 7, Proceedings of the International Symposium on chemical synthesis of Nucleic Acids, held in Egerstorf, GFR on 5th-8th May 1980, edited by H. Köster, IRL Press August 1980 LONDON (GB) S.A. NARANG et al.: "Synthesis of the human insulin gene. Part IV. New synthetic deoxyribooligonucleotide adaptors and primer for DNA cloning and sequence analysis" pages 377-385**

## Description

Thaumatin is a protein originating from the arils of the fruit of *Thaumatococcus daniellii*. Thaumatin is, on a weight basis, 1600 times sweeter than sucrose and on a molecular basis $10^5$ times sweeter than sucrose. In Western society overconsumption of sugar causes a number of health problems. Therefore, many attempts have been made to substitute low caloric sweeteners for sugar. However, several of these have recently been prohibited in view of possible side-effects. There is therefore a need for a natural low caloric sweetener and for an economical process of producing such a sweetener. Recent advances in molecular biology have enabled the introduction of structural genes coding for specific eukaryotic proteins into microbial host cells and expressing said genes in the transformed host cells, thereby producing the desired protein.

Many genes of eukaryotic origin which in their natural state encode proteins in their unprocessed forms cannot be applied directly in recombinant DNA molecules because natural genes contain DNA sequences called introns, which are not contained in the messenger RNA (mRNA). The information located on these introns is removed in eukaryotic cells before the translation process of the mRNA. As far as Applicants are aware, bacteria are unable to excise such introns at RNA level and therefore it is necessary to remove the genetic information located on these introns at RNA level before the natural gene of eukaryotes can be used in prokaryotic host cells.

In microbial host cells which have the capability of excising introns at mRNA level, the natural genes can in principle be applied, provided that they are brought under control of regulons that are effective in said microbial host cells.

In the present invention use is made of recombinant DNA techniques that introduce the genetic information of eukaryotic, particularly of plant, origin, in such a state that expression occurs effectively in microbial, particularly in bacterial host cells.

For a better understanding of the invention the most important terms used in the description will be defined:

A regulon is a DNA sequence consisting of a promotor and operator region.

Structural genes are DNA sequences which encode through a template (mRNA) a sequence of amino acids characteristic of a specific polypeptide.

A promoter is a DNA sequence within the regulon to which RNA polymerase binds for the initiation of the transcription.

An operator is a DNA sequence within the regulon to which a repressor protein may bind, thus preventing RNA polymerase from binding to the adjacent promoter.

An inducer is a substance which deactivates a repressor protein, freeing the operator and permitting RNA polymerase to bind to the promoter and start transcription.

By mature thaumatin is meant any of the allelic forms of the fully processed protein of which one is given in Fig. 1.

Cloning vehicle. A non-chromosomal double-stranded DNA, plasmid or phage, comprising a DNA sequence (intact replicon) that allows self-replication after transformation into suitable host cells.

Phage or bacteriophage. Bacterial virus which can replicate in a suitable bacterial host cell.

Reading frame. The grouping of triplets of nucleotides (codons) into such a frame that at mRNA level a proper translation of the codons into the polypeptide takes place.

Transcription. The process of producing RNA from a gene.

Translation. The process of producing a polypeptide from mRNA.

Expression. The process undergone by a structural gene to produce a polypeptide. It is a combination of many processes, including at least transcription and translation.

By mature thaumatin gene is meant the double-stranded DNA sequence having exactly the same information content (sequence of codons) as that part of the messenger RNAs coding for various allelic forms of thaumatin in their fully processed (mature) form. For reasons of convenience only one strand of ds DNA is given in the text and figures.

Thus the present invention provides a new process of producing mature thaumatin, namely by means of bacteriological production. Therefore the present invention in the first place provides a DNA sequence selected from the group consisting of the various allelic forms of the mature thaumatin gene given in Fig. 2, namely:

32 polynucleotides, one of which has the composition given in Fig. 1 and the other 31 have the same composition as given in Fig. 1 except that they contain one or more variations in nucleotides 138 (either G or C), 187 (either C or A), 199 and 200 (either CG or AA), 227 (either A or G) and 337 (either G or A).

The DNA sequence having the polynucleotide composition according to Fig. 1 was found by the procedure described below in the specification yielding a plasmid pUR100 containing the DNA sequence of Fig. 1, whereby arils of the fruits of *Thaumatococcus daniellii* were used as the starting material. This DNA sequence is the same as the DNA sequence indicated with wild type in Fig. 2. The DNA sequence containing all five variations in Fig. 2 is the one corresponding to the thaumatin described by Iyengar et al. in Eur. J. Biochem. *96* (1979), 193—204. The other 30 DNA sequences differ in only one to four places from the wild type DNA sequence of Fig. 2.

The invention further provides recombinant plasmids comprising

(i) a DNA sequence as claimed in claim 1, and
(ii) an inducible or constitutive regulon which regulates the expression of said DNA sequences.

A preferred inducible regulon consists of a double lac UV5 system as described by D. V. Goeddel et al., Nature *281*, 544—548 (1979).

A particularly preferred recombinant plasmid containing a double lac UV5 regulon and a mature thaumatin gene is obtainable by preparation from
(a) plasmid pUR201,
(b) one of the 32 thaumatin-encoding DNA sequences of Fig. 2, and
(c) the *Eco*RI-linker

$$(5')pCAT(N)_nGAATTC(N')_nATG_{OH}(3'),$$

wherein n=0, 1, 2 or 3 and N and N' are any of the nucleotides A, T, G or C, with the proviso that in the double-stranded structure N and N' are such that a rotational symmetrical structure is present, whereby plasmid pUR201 is obtainable by
(a.1) cleaving plasmid pBR322 with *Eco*RI,
(a.2) cleaving plasmid pKB268 with *Eco*Ri and isolating the DNA fragment encoding the double lac (UV5) regulon,
(a.3) ligating the regulon and the *Eco*RI-cleaved pBR322,
(a.4) selectively cleaving the resulting plasmid with *Eco*RI, in the presence of RNA polymerase, followed by S1 nuclease treatment and subsequent ligation with T4 ligase, resulting in a plasmid (pUR201) in which the *Eco*RI site most distant from the *Hin*dIII site in the tetracycline resistance gene is selectively removed.

Another preferred inducible regulon is a constituent of the tryptophan system described by F. Lee et al., J. Mol. Biol. *121*, 193—217 (1978) and K. Bertrand et al., Science *189*, 22—26 (1975). Applicants have modified this tryptophan system to obtain a more adequate system according to Fig. 3. In this modified system the information coding for the trp attenuator protein is eliminated while maintaining the ribosome binding site.

Thus the invention also provides recombinant plasmids comprising a DNA sequence encoding mature thaumatin according to the invention as described above and a modified tryptophan system regulating the expression of the structural genes, which modified tryptophan system is obtainable by restriction endonuclease *Hin*fI cleavage of trp ED5, followed by subsequent treatment of the DNA fragment of about 510 base pairs so obtained with restriction endonuclease *Taq*I, in the presence of *E. coli* RNA polymerase, while protecting the *Taq*I site in the trp regulon, yielding a fragment containing 234 base pairs comprising the trp regulon, then treating the latter fragment with S1 nuclease, blunt-end ligation with the *Eco*RI-linker

$$(5')pGGAATTCC_{OH}(3'),$$

cutting with *Eco*RI and subsequently cloning in the *Eco*RI site of pBR322.

A particularly preferred form of such a plasmid is obtainable by preparation from
(a) plasmid pUR301
(b) one of the 32 thaumatin-encoding DNA sequences of Fig. 2, and
(c) the *Eco*RI linker

$$(5')pCAT(N)_nGAATTC(N')_nATG_{OH}(3'),$$

wherein n=0, 1, 2 or 3 and N and N' are any of the nucleotides A, T, G or C, with the proviso that in the double-stranded structure N and N' are such that a rotational symmetrical structure is present, whereby plasmid pUR301 is obtainable by:
selectively cleaving the pBR322-derived plasmid containing the modified tryptophan regulon described in claim 5 with *Eco*RI, in the presence of ethidium bromide, followed by S1 nuclease treatment and subsequent ligation with T4 ligase, resulting in a plasmid (pUR301) in which the *Eco*RI site most distant from the *Hin*dIII site in the tetracycline resistance gene is selectively removed.

The recombinant plasmid according to the invention may comprise DNA sequences consisting of a modified promoter/ribosome-binding site of gene VIII of bacteriophage M13, *fd* of *fl* [P.M.G.F. van Wesenbeek et al., Gene *11*, 129—148 (1980)], which, as far as Applicants are aware, were never used before for the expression of eukaryotic genes.

Thus the invention further provides recombinant plasmids comprising a DNA sequence encoding mature thaumatin according to the invention as described above and a modified M13 gene VIII system comprising double-stranded DNA consisting of nucleotides 1128—1291 described by Van Wezenbeek et al. A particularly preferred form of such a plasmid is obtainable by preparation from
(a) plasmid pUR401,
(b) one of the 32 thaumatin-encoding DNA sequences of Fig. 2, and
(c) the *Eco*RI-linker

$$(5')pCAT(N)_nGAATTC(N')_nATG_{OH}(3'),$$

wherein n=0, 1, 2 or 3 and N and N' are any of the nucleotides A, T, G or C, with the proviso that in the double-stranded structure N and N' are such that a rotational symmetrical structure is present,
whereby plasmid pUR401 is obtained by
(a.1) the blunt-end ligation of the *Eco*RI-linker

$$(5')pGGAATTCC_{OH}(3')$$

with the modified M13 gene VIII regulon consisting of polynucleotide 1128—1293 (which is obtainable by subsequent treatment of RFM13 DNA with *Taq*I and *Hae*III, repair treatment of the *Taq*I site with *E. coli* DNA polymerase, partly digesting the fragment with *Mnl*I, followed by successive treatment with T4 DNA polymerase and S1 nuclease)
(a.2) treatment of the resulting fragment of (a.1)

with *Eco*RI, followed by ligation with *Eco*RI-cleaved pBR322,

(a.3) selectively cleaving the pBR322-derived plasmid containing the modified M13 gene VIII regulon with *Eco*RI, in the presence of ethidium bromide, followed by S1 nuclease treatment and subsequent ligation with T4 ligase, resulting in a plasmid (pUR401) in which the *Eco*RI site most distant from the *Hin*dIII site in the tetracycline resistance gene is selectively removed.

In the recombinant plasmids according to the invention the regulon may be either directly linked to the structural gene or indirectly through a novel start codon and *Eco*RI-site-containing DNA linker having the nucleotide sequence

$$(5')pCAT(N)_nGAATTC(N')_nATG_{OH}(3),$$

wherein n=0, 1, 2 or 3, and

N and N′ are any of the nucleotides A, T, G or C, with the proviso that in the double-stranded structure N and N′ are such that a rotational symmetrical structure is present.

By a rotational symmetrical structure is meant that where N is e.g. represented by A, N′ should be represented by the complementary base T.

Thus one embodiment of the invention is a process for the preparation of recombinant plasmids according to the invention as described above, in which process a DNA sequence encoding mature thaumatin as defined above is linked to the *Eco*RI site of an inducible or constitutive regulon which regulates the expression of said DNA sequence, characterized in that

(a) the 5′-end of said DNA sequence is coupled with a linker having the nucleotide sequence

$$(5')pCAT(N)_nGAATTC(N')_nATG_{OH}(3'),$$

wherein n=0, 1, 2 or 3, and N and N′ are any of the nucleotides A, T, G or C, with the proviso that in the double-stranded structure N and N′ are such that a rotational symmetrical structure is present,

(b) the coupling product is treated with *Eco*RI, and

(c) the *Eco*RI-treated coupling product is coupled with the *Eco*RI-treated regulon.

In some instances it turned out that the yield of expression improves when the sequence AATT between the regulon and the structural gene has been eliminated.

An embodiment of the invention coming closer to the commercial production of mature thaumatin is a bacterial culture of *E. coli* cells containing any one of the recombinant plasmids according to the invention as described above with or without the AATT sequence originating from the ATG- and *Eco*RI-site-containing linker described above, situated between the regulon and the structural gene of the recombinant plasmid. Preferred bacterial cultures contain a recombinant plasmid selected from the plasmids belonging to the pUR520 family, the pUR530 family or to the pUR540 family, containing a mature thaumatin-encoding DNA sequence and a double lac UV5 regulon, a modified tryptophan regulon or a modified M13 gene VIII regulon, respectively.

Finally the invention provides a process for producing mature thaumatin which comprises incorporating a recombinant plasmid according to the invention in *E. coli* cells, culturing the transformed cells and isolating the thaumatin produced by said cells.

The microbial cloning vehicles containing the structural genes encoding various allelic forms of the mature (fully processed) thaumatin were obtained and various thaumatins were produced by performing a number of steps, the most essential of which are:

(1) isolation and purification of the messenger RNA (mRNA) of thaumatin from *Thaumatococcus daniellii*;
(2) conversion of this mRNA into double-stranded DNA (ds DNA);
(3) construction of ds DNA having a poly-dC tail;
(4) incorporation of the ds DNA-poly-dC molecules in endonuclease *Pst*I-cleaved, and poly-dG-tailed plasmid pBR322 DNA;
(5) transformation and clone selection (yielding pUR100);
(6) determination of the nature of the insert by RNA/DNA hybridization and *in vitro* translation;
(7) double-checking the nature of the inserts by DNA- and RNA-sequence analysis;
(8) producing DNA encoding the mature fully processed thaumatin (see Fig. 4);
(9) construction of plasmids comprising specific transcription regulating DNA sequences, and chemical synthesis of DNA-linkers and -primers;
(10) construction of plasmids comprising a constitutive or inducible regulon and the ligated thaumatin gene; and transformation of *E. coli* with said plasmids;
(11) culturing of *E. coli* cells containing said recombinant plasmids and detection and isolation of the thaumatin.

Steps (1)—(8) yielded a DNA sequence, the structure of which is given in Fig. 1, followed by a stop codon as shown in Fig. 2 and Fig. 4, last line.

The sequences of claim 1 can be prepared synthetically or semi-synthetically, for instance using the corresponding steps mentioned above for the preparation of partial sequences thereof.

The following detailed description will illustrate the invention.

1a. Construction of plasmid pUR201

A fragment containing 285 base pairs comprising the double lac regulon (lac UV5) was obtained by restriction endonuclease *Eco*RI cleavage of pKB268, (K. Backman and M. Ptashne, Cell *13*, 65—71 (1978)). This fragment was ligated in the *Eco*RI site of pBR322 DNA. Plasmid DNA

with the lac regulon in the right orientation (Fig. 5) was partly cleaved by *Eco*RI in the presence of *E. coli* RNA polymerase. The *Eco*RI cleavage site most distant from the restriction endonuclease *Hin*dIII cleavage site was preferentially attacked. The linearized DNA was treated with S1 nuclease, purified by agarose gel electrophoresis, circularized by ligation with T4 DNA-ligase and subsequently used to transform *E. coli*. From the tetracycline-resistant transformants pUR201 with the correct structure (Fig. 5) was obtained.

1b. Construction of plasmid pUR301

A DNA fragment of about 510 base pairs was obtained by restriction endonuclease *Hin*fI cleavage of ptrpED5, (R. A. Hallewell and S. Emtage, Gene *9*, 27—47 (1980)). This fragment was cleaved with restriction endonuclease *Taq*I in the presence of *E. coli* RNA polymerase. The *Taq*I site in the trp regulon (described by K. Bertrand et al., Science *189*, 22—26 (1975) and F. Lee et al., J. Mol. Biol. *121*, 193—217 (1978)) was selectively protected, thus yielding a fragment containing 234 base pairs comprising the trp regulon (Fig. 3). This fragment was then treated with S1 nuclease, blunt-end ligated with the *Eco*RI-linker

$$(5')pGGAATTCC_{OH}(3'),$$

cut with *Eco*RI and subsequently cloned in the *Eco*RI-site of pBR322.

Plasmid pUR300 with the trp regulon in the correct orientation (Fig. 3) was isolated. The *Eco*RI-cleavage site most distant from the *Hin*dIII site was removed by partial cleavage of pUR300 DNA by *Eco*RI in the presence of ethidium bromide, and S1 nuclease treatment. Linear DNA molecules were recircularized by T4 DNA ligase. From the tetracycline-resistant transformants pUR301 with the structure as outlined in Fig. 3 was obtained.

1c. Construction of plasmid pUR401

A fragment containing 270 base pairs (DNA sequence 1128—1397) was obtained by digestion of RF M13 DNA (see P. M. G. F. van Wezenbeek et al., Gene *11*, 129—148 (1980)), with the restriction endonuclease *Taq*I and *Hae*III and the *Taq*I site was made blunt-ended by a repair reaction with *E. coli* DNA polymerase; the fragment was subsequently partly digested with restriction enzyme *Mnl*I. The partial products were treated with successive actions of T4 DNA polymerase and S1 nuclease and subsequently blunt-end ligated with the *Eco*RI-linker

$$(5')pGGAATTCC_{OH}(3'),$$

then treated with *Eco*RI and ligated in the *Eco*RI site of the pBR322. By restriction enzyme analysis and DNA sequence analysis a plasmid was obtained in which the *Eco*RI cleavage site was located just beyond the ribosome-binding site of the M13 gene VIII DNA sequence. Applicants have found that the plasmids having the M13 regulon from nucleotide 1128 to nucleotides 1291 to 1297 were appropriate regulons for expression. The *Eco*RI cleavage site most distant from the *Hin*dIII site was removed essentially as described for pUR301. The complete construction of pUR401 is outlined in Fig. 6.

1d. Chemical synthesis of linkers and primers

The synthesis of oligodeoxynucleotides is carried out through coupling of 5' - O - levulinyl-deoxynucleoside - 3' - O - 2,2,2 - trichloro-ethyl - 2 - chorophenyl phosphates with deoxy-nucleoside - 3' - O - 2,2,2 - trichloroethyl - 2 - chlorophenyl phosphates. This method, which is known as the phosphotriester method (described by J. F. M. de Rooij et al., Recl. Trav. Chim. Pays-Bas *98*, 537—548 (1979)), involves splitting off the trichloroethyl group by active zinc, followed by the actual coupling reaction with the help of 2,4,6 - triisopropylbenzenesulphonyl - 3 - nitro - 1,2,4 - triazole. The amino groups in deoxyadenosine, deoxycytidine and deoxyguano-sine are protected by a benzoyl group, a 4-methoxybenzoyl group and a benzoyl group respectively. For the protection of the 3'-hydroxy function of the terminal nucleoside the benzoyl group is used. In the last step all protecting groups are removed through reaction with tetrabutylammonium fluoride and concentrated aqueous ammonia, respectively.

An example of the construction of the linker

$$(5')pCATGAATTCATG_{OH}(3')$$

is given in Fig. 7.

2. Construction of expression plasmids with the gene of Fig. 4, last line, containing the sequence for mature thaumatin of Fig. 1, followed downstream by the stop codon TGA under transcription control of the double lac UV5 regulon (pUR520 family), the trp regulon (pUR530 family) and the M13 or *fl* or *fd* gene VIII regulon (pUR540 family) and transformation of *E. coli* with said plasmids.

The thaumatin encoding DNA fragment of Fig. 4, last line was blunt-end ligated with the synthetic *Eco*RI-linkers

$$(5')pCAT(N)_nGAATTC(N')_nATG_{OH}(3')$$

(wherein n=0, 1, 2 or 3 and N and N' are any of the nucleotides A, T, G or C, with the proviso that in the double-stranded structure N and N' are such that a rotational symmetrical structure is present) with T4 DNA ligase, cleaved with *Eco*RI and subsequently ligated in the *Eco*RI-cleavage site of the plasmids pUR201, pUR301 and pUR401, and recombinant plasmids with the thaumatin encoding insert in the orientations as illustrated in Fig. 8 were isolated after transformation of *E. coli* and selection of tetracycline-resistant transformants. In the above-described plasmids the AATT sequence originating from the chemically synthesized

linkers could be deleted by cleavage of the plasmids with *Eco*RI in the presence of ethidium bromide; linear partials were isolated by agarose gel electrophoresis, treated with S1 nuclease and recircularized by T4 DNA ligate action.

Plasmids obtained after deletion of AATT were detected by restriction enzyme analysis.

3. Culturing of *E. coli* cells containing said recombinant plasmids and detection of the thaumatin

*E. coli* cells containing plasmids of the pUr520 or pUR530 or pUR540 families with or without the AATT sequence in the linker between the regulon and the mature thaumatin gene(s) in the correct orientation and reading frame were cultured under conditions most suitable for their growth—these culturing conditions vary with the type of plasmid present in the cells—but always in the presence of an appropriate antibiotic to maintain selection pressure. Under these conditions the cells containing either plasmids of the pUR520 or pUR530 or pUR540 families produced considerable amounts of mature thaumatin.

The presence of the protein thaumatin was demonstrated qualitively by S.D.S. electrophoresis and by physiological tests on their sweetness and quantitatively by the enzyme linked immuno sorbent assay (ELISA).

**Claims for the Contracting States: BE, CH, DE, FR, GB, IT, LI, NL, SE**

1. A DNA sequence selected from the group consisting of the various allelic forms of the mature thaumatin gene given in Fig. 2, namely: 32 polynucleotides, one of which has the composition given in Fig. 1 and the other 31 have the same composition as given in Fig. 1 except that they contain one or more variations in nucleotides 138 (either G or C), 187 (either C or A), 199 and 200 (either CG or AA), 227 (either A or G) and 337 (either G or A).

2. Recombinant plasmids comprising

(i) a DNA sequence as claimed in claim 1, and
(ii) an inducible or constitutive regulon which regulates the expression of said DNA sequences.

3. Recombinant plasmids according to claim 2, comprising an inducible regulon consisting of a double lac UV5 system regulating the expression of the structural genes.

4. Recombinant plasmid according to claim 3, obtainable by preparation from

(a) plasmid pUR201,
(b) one of the 32 thaumatin-encoding DNA sequences of Fig. 2, and
(c) the *Eco*RI-linker

$$(5')pCAT(N)_nGAATTC(N')_nATG_{OH}(3'),$$

wherein n=0, 1, 2 or 3 and N and N' are any of the nucleotides A, T, G or C, with the proviso that in the double-stranded structure N and N' are such that a rotational symmetrical structure is present,

whereby plasmid pUR201 is obtainable by
(a.1) cleaving plasmid pBR322 with *Eco*RI,
(a.2) cleaving plasmid pKB268 with *Eco*RI and isolating the DNA fragment encoding the double lac (UV5) regulon,
(a.3) ligating the regulon-containing DNA fragment and the *Eco*RI-cleaved pBR322
(a.4) selectively cleaving the resulting plasmid with *Eco*RI in the presence of RNA polymerase, followed by S1 nuclease treatment and subsequent ligation with T4 ligase resulting in a plasmid (pUR201), in which the *Eco*RI site most distant from the *Hin*dIII site in the tetracycline resistance gene is selectively removed.

5. Recombinant plasmids according to claim 2, comprising a modified tryptophan system regulating the expression of the structural genes, which modified tryptophan system is obtainable by restriction endonucleuse *Hin*fI cleavage of trpED5, followed by subsequent treatment of the DNA fragment of about 510 base pairs so obtained with restriction endonuclease *Taq*I in the presence of *E. coli* RNA polymerase, while protecting the *Taq*I site in the trp regulon, yielding a fragment containing 234 base pairs comprising the trp regulon, then treating the latter fragment with S1 nuclease, blunt-end ligation with the *Eco*RI-linker

$$(5')pGGAATTCC_{OH}(3'),$$

cutting with *Eco*RI and subsequently cloning in the *Eco*RI site of pBR322.

6. Recombinant plasmid according to claim 5, obtainable by preparation from

(a) plasmid pUR301
(b) one of the 32 thaumatin-encoding DNA sequences of Fig. 2, and
(c) the *Eco*RI-linker

$$(5')pCAT(N)_nGAATTC(N')_nATG_{OH}(3'),$$

wherein n=0, 1, 2 or 3 and N and N' are any of the nucleotides A, T, G or C, with the proviso that in the double-stranded structure N and N' are such that a rotational symmetrical structure is present,

whereby plasmid pUR301 is obtainable by selectively cleaving the PBR322-derived plasmid containing the modified tryptophan regulon described in claim 5 with *Eco*RI in the presence of ethidium bromide, followed by S1 nuclease treatment and subsequent ligation with T4 ligase resulting in a plasmid (pUR301), in which the *Eco*RI site most distant from the *Hin*dIII site in the tetracycline resistance gene is selectively removed.

7. Recombinant plasmids according to claim 2, comprising a modified M13 gene VIII system regulating the expression of the structural genes, which modified M13 gene VIII system comprises double-stranded DNA consisting of nucleotides 1128—1291.

8. Recombinant plasmid according to claim 7, obtainable by preparation from

(a) plasmid pUR401,

(b) one of the 32 thaumatin-encoding DNA sequences of Fig. 2, and

(c) the *Eco*RI-linker

$$(5')pCAT(N)_nGAATTC(N')_nATG_{OH}(3'),$$

wherein n=0, 1, 2 or 3 and N and N'are any of the nucleotides A, T, G or C, with the proviso that in the double-stranded structure N and N' are such that a rotational symmetrical structure is present,

whereby plasmid pUR401 is obtained by

(a.1) blunt-end ligation of the *Eco*RI-linker

$$(5')pGGAATTCC_{OH}(3')$$

with the modified M13 gene VIII regulon consisting of polynucleotide 1128—1293 (which is obtainable by subsequent treatment of RFM13 DNA with *Taq*I and *Hae*III, repair treatment of the *Taq*I site with *E. coli* DNA polymerase, partly digesting the fragment with *Mnl*I, followed by successive actions of T4 DNA polymerase and S1 nuclease),

(a.2) treatment of the resulting fragment of (a.1) with *Eco*RI, followed by ligation with *Eco*RI-cleaved pBR322,

(a.3) selectively cleaving the pBR322-derived plasmid containing the modified M13 gene VIII regulon with *Eco*RI in the presence of ethidium bromide, followed by S1 nuclease treatment and subsequent ligation with T4 ligase resulting in a plasmid (pUR401), in which the *Eco*RI site most distant from the *Hin*dIII site in the tetracycline resistance gene is selectively removed.

9. A process for the preparation of recombinant plasmids as claimed in claim 2, in which process a DNA sequence as claimed in claim 1 is linked to the *Eco*RI site of a regulon as described in claim 2, characterized in that

(a) the 5'-end of said DNA sequence is coupled with a linker having the nucleotide sequence

$$(5')pCAT(N)_nGAATTC(N')_nATG_{OH}(3'),$$

wherein n=0, 1, 2 or 3, and N and N' are any of the nucleotides A, T, G or C, with the proviso that in the double-stranded structure N and N' are such that a rotational symmetrical structure is present,

(b) the coupling product is treated with *Eco*RI, and

(c) the *Eco*RI-treated coupling product is coupled with the *Eco*RI-treated regulon.

10. A bacterial culture comprising *E. coli* cells containing any one of the recombinant plasmids as claimed in claims 2—8 with or without the AATT sequence originating from the linker as described in claim 9 situated between the regulon and the structural gene of the recombinant plasmid.

11. A bacterial culture according to claim 10, in which the recombinant plasmid is selected from the group consisting of the plasmids as claimed in claims 4, 6 and 8.

12. A process for producing mature thaumatin which comprises incorporating a recombinant plasmid as claimed in any one of claims 2—8 in *E. coli* cells, culturing the transformed cells and isolating the thaumatin produced by said cells.

**Claims for the Contracting State: AT**

1. A process for producing DNA sequences encoding the various allelic forms of mature thaumatin, characterized in that:

a DNA sequence selected from the group consisting of the various allelic forms of the mature thaumatin gene given in Fig. 2, namely:

32 polynucleotides, one of which has the composition given in Fig. 1 and the other 31 have the same composition as given in Fig. 1 except that they contain one or more variations in nucleotides 138 (either G or C), 187 (either C or A), 199 and 200 (either CG or AA), 227 (either A or G) and 337 (either G or A), is prepared synthetically or semi-synthetically, preferably by using the steps (1)—(8) mentioned in the specification for the partial preparation of partial sequences of the mature thaumatin gene.

2. A process for producing recombinant plasmids, characterized in that recombinant plasmids are produced which comprise DNA sequences encoding any of the various allelic forms of mature thaumatin as described in claim 1 and an inducible or constitutive regulon which regulates the expression of said DNA sequences.

3. A process according to claim 2, characterized in that a plasmid of the pUR520 family containing a double lac UV5 system as the regulon is produced.

4. A process according to claim 2, characterized in that a plasmid of the pUR530 family containing a modified tryptophan system as the regulon is produced, which modified tryptophan system is obtainable by restriction endonuclease *Hin*fI cleavage of trpED5, followed by subsequent treatment of the DNA fragment of about 510 base pairs so obtained with restriction endonuclease *Taq*I in the presence of *E. coli* RNA polymerase, while protecting the *Taq*I site in the trp regulon, yielding a fragment containing 234 base pairs comprising the trp regulon, then treating the latter fragment with S1 nuclease, blunt-end ligation with the *Eco*RI-linker

$$(5')pGGAATTCC_{OH}(3'),$$

cutting with *Eco*RI and subsequently cloning in the *Eco*RI site of pBR322.

5. A process according to Claim 2, characterized in that a plasmid of the pUR540 family containing polynucleotide 1128—1291 of the M13 gene VIII system as the regulon is produced.

6. A process for the preparation of recombinant plasmids as claimed in claim 2, in which process a DNA sequence as claimed in claim 1 is linked to

the *Eco*RI site of a regulon as described in claim 2, characterized in that

(a) the 5'-end of said DNA sequence is coupled with a linker having the nucleotide sequence

$$(5')pCAT(N)_nGAATTC(N')_nATG_{OH}(3'),$$

wherein n=0, 1, 2 or 3, and N and N' are any of the nucleotides A, T, G or C, with the proviso that in the double-stranded structure N and N' are such that a rotational symmetrical structure is present,

(b) the coupling product is treated with *Eco*RI, and

(c) the *Eco*RI-treated coupling product is coupled with the *Eco*RI-treated regulon.

7. A process for producing mature thaumatin, characterized in that

(1) plasmids produced according to a process claimed in any one of the preceding claims 2—6 are incorporated in *E. coli* cells;
(2) transformed cells are cultivated; and
(3) thaumatin produced by said cells is isolated.

8. A process according to claim 7, characterized in that a plasmid produced according to a process as claimed in claims 3, 4 and 5, with or without the AATT sequence originating from the linker as described in claim 6 situated between the regulon and the mature thaumatin gene of the recombinant plasmid, is incorporated in *E. coli* cells.

**Patentansprüche für die Vertragsstaaten: BE, CH, DE, FR, GB, IT, LI, NL und SE**

1. Eine DNS-Sequenz, ausgewählt aus der Gruppe von verschiedenen allelen Formen des vollausgereiften Thaumatingens, angegeben in Fig. 2, nämlich: 32 Polynukleotiden, wovon eines die in Fig. 1 angegebene Zusammensetzung hat und die anderen 31 die gleiche Zusammensetzung wie in Fig. 1 mit der Ausnahme haben, daß sie eine oder mehrere Variationen in den Nukleotiden 138 (entweder G oder C), 187 (entweder C oder A), 199 und 200 (entweder CG oder AA), 227 (entweder A oder G) und 337 (entweder G oder A) enthalten.

2. Rekombinante Plasmide, die enthalten:

(I) eine DNS-Sequenz gemäß Anspruch 1 und
(II) ein induzierbares oder konstitutives Regulon, das die Expression der genannten DNS-Sequenzen reguliert.

3. Rekombinante Plasmide nach Anspruch 2, dadurch gekennzeichnet, daß sie ein induzierbares Regulon enthalten, das aus einem Doppel-lac-$UV_5$-System besteht, das die Expression der Strukturgene reguliert.

4. Rekombinantes Plasmid nach Anspruch 3, erhältlich durch Herstellung aus

(a) dem Plasmid pUR201,

(b) einen der 32 Thaumatin-kodierenden DNS-Sequenzen von Fig. 2 und

(c) dem *Eco*RI-Linker

$$(5')pCAT(N)_nGAATTC(N')_nATG_{OH}(3'),$$

worin n=0, 1, 2 oder 3 ist und N und N' irgendeines der Nukleotide A, T, G oder C sind, was unter der Bedingung gilt, daß die Doppelstrangstruktur N und N' derartig ist, daß eine rotationssymmetrische Struktur vorliegt, wobei das Plasmid pUR201 erhältlich ist durch

(a.1) Spalten des Plasmids pBR322 mit *Eco*RI,

(a.2) Spalten des Plasmids pKB268 mit *Eco*RI und Isolieren des DNS-Fragments, das das Doppel-lac-($UV_5$)-Regulon kodiert,

(a.3) Verknüpfen des Regulon-enthaltenden DNS-Fragments und des *Eco*RI-gespaltenen pBR322,

(a.4) selektives Spalten des erhaltenen Plasmids mit *Eco*RI in Gegenwart von RNS-Polymerase, gefolgt durch S1-Nuklease-Behandlung und nachfolgende Verknüpfung mit T4-Ligase, was zu einem Plasmid (pUR201) führt, in dem die *Eco*RI-Stelle mit dem größten Abstand zue *Hin*dIII-Stelle im Tetracyclinresistenz - Gen selektiv entfernt wird.

5. Rekombinante Plasmide nach Anspruch 2, dadurch gekennzeichnet, daß sie ein modifiziertes Tryptophan-System enthalten, das die Expression der Struktur-Gene reguliert, wobei das modifizierte Tryptophan-System durch Restriktionsendonuklease - *Hinf*I - Spaltung von $trpEd_5$ erhältlich ist, gefolgt durch nachfolgende Behandlung des DNS-Fragments von etwa 510 Basenpaaren, derartig mit der Restriktionsendonuklease *Taq*I in Gegenwart der *E. coli*-RNS-Polymerase erhalten, während die *Taq*I-Stelle in dem trp-Regulon geschützt ist, was zu einem Fragment führt, das 234 Basenpaare mit dem trp-Regulon enthält, darauf Behandeln des letzteren Fragmentes mit S1-Nuklease, Ligation der glatten Enden mit dem *Eco*RI-Linker

$$(5')pGGAATTCC_{OH}(3'),$$

Schneiden mit *Eco*RI und nachfolgendes Klonieren in der *Eco*RI-Stelle des pBR322.

6. Rekombinantes Plasmid nach Anspruch 5, erhältlich durch die Herstellung aus

(a) dem Plasmid pUR301,

(b) einer von den 32 Thaumatin-kodierenden DNS-Sequenzen der Fig. 2, und

(c) dem *Eco*RI-Linker

$$(5')pCAT(N)_nGAATTC(N')_nATG_{OH}(3'),$$

worin n=0, 1, 2 oder 3 ist und N und N' irgendwelche der Nukleotide A, T, G oder C sind, was unter der Bedingung gilt, daß in der Doppelstrangstruktur N und N' derartig sind, daß eine rotationssymmetrische Struktur vorliegt, wodurch das Plasmid pUR301 erhältlich ist durch selektives Spalten des pBR322-abgeleiteten Plasmids, das das in Anspruch 5 beschriebene modifizierte Tryptophan-Regulon enthält, mit *Eco*RI in Gegenwart von Ethidiumbromid, gefolgt durch S1-Nuklease-Behandlung und nachfolgende Ligation mit T4-Ligase, was zu

einem Plasmid (pUR301) führt, in dem die *Eco*RI-Stelle mit dem größten Abstand zur *Hin*dIII-Stelle im Tetracyclinresistenzgen selektiv entfernt wird.

7. Rekombinant Plasmide nach Anspruch 2, dadurch gekennzeichnet, daß sie ein modifiziertes M13-Gen-VIII-System enthalten, das die Expression der Strukturgene reguliert, wobei das modifizierte M13-Gen-VIII-System aus den Nukleotiden 1128—1291 bestehende Doppelstrang-DNS enthält.

8. Rekombinantes Plasmid nach Anspruch 7, erhältlich durch die Herstellung aus

(a) dem Plasmid pUR401,

(b) einer von 32 Thaumatin-kodierenden DNS-Sequenzen der Fig. 2 und

(c) dem *Eco*RI-Linker

$$(5')pCAT(N)_nGAATTC(N')_nATG_{OH}(3'),$$

worin n=0, 1, 2 oder 3 ist und N und N' irgendwelche der Nukleotide A, T, G oder C sind, was unter der Bedingung gilt, daß in der Doppelstrangstruktur N und N' derartig sind, daß eine rotationssymmetrische Struktur vorliegt, wodurch das Plasmid pUR401 erhalten wird durch

(a.1) Ligation der glatten Enden des *Eco*RI-Linkers

$$(5')pGGAATTCC_{OH}(3')$$

mit dem modifizierten M13-Gen-VIII-Regulon, das aus dem Polynukleotid 1128—1293 besteht (welches erhältlich ist durch nachfolgende Behandlung von RFM13 DNS mit *Taq*I und *Hae*III, Ausbesserung der *Taq*I-Stelle mit der *E. coli*-DNS-Polymerase, teilweises Zerlegen des Fragments mit *Mn*/I, gefolgt durch nachfolgende Einwirkungen der T4-DNS-Polymerase und S1-Nuklease),

(a.2) Behandlung der erhaltenen Fragments von (a.1) mit *Eco*RI, gefolgt durch Ligation mit *Eco*RI-gespaltenem pBR322,

(a.3) selektives Spalten des pBR322-abgeleiteten Plasmids, das das modifizierte M13-Gen-VIII-Regulon enthält, mit *Eco*RI in Gegenwart von Ethidiumbromid, gefolgt durch S1-Nuklease-Behandlung und nachfolgende Ligation mit T4-Ligase, was zu einem Plasmid (pUR401) führt, in dem die *Eco*RI-Stelle mit dem größten Abstand zur *Hin*dIII-Stelle im Tetracyclinresistenzgen selektiv entfernt wird.

9. Verfahren zur Herstellung von rekombinanten Plasmiden gemäß Anspruch 2, bei dem eine in Anspruch 1 beanspruchte DNS-Sequenz mit der *Eco*RI-Stelle eines im Anspruch 2 beschriebenen Regulons verbunden wird dadurch gekennzeichnet, daß

(a) das 5'-Ende der genannten DNS-Sequenz mit einem Linker gekuppelt wird, der die Nukleotid-Sequenz

$$(5')pCAT(N)_nGAATTC(N')_nATG_{OH}(3')$$

enthält, worin n=0, 1, 2 oder 3 ist und N und N' irgendwelche der Nukleotide A, T, G oder C sind, was unter der Bedingung gilt, daß in der Doppelstrangstruktur N und N' derartig sind, daß eine rotationssymmetrische Struktur vorliegt,

(b) das Kupplungsprodukt mit *Eco*RI behandelt wird und

(c) das *Eco*RI-behandelte Kupplungsprodukt mit dem *Eco*RI-behandelten Regulon gekuppelt wird.

10. Bakterienkultur mit einem Gehalt an *E. coli*-Zellen, die irgendeines, der rekombinanten Plasmide, wie in den Ansprüchen 2 bis 8 beansprucht, mit oder ohne AATT-Sequenz enthält, die auf den Linker, wie er in Anspruch 9 beschrieben wird, zurückgeht, gelegen zwischen dem Regulon und dem Strukturgen des rekombinanten Plasmids.

11. Bakterienkultur nach Anspruch 10, dadurch gekennzeichnet, daß das rekombinante Plasmid aus der aus den in den Ansprüchen 4, 6 und 8 bestehenden Gruppe von Plasmiden ausgewählt worden ist.

12. Verfahren zu Herstellung von reiferm Thaumatin, dadurch gekennzeichnet, daß *E. coli*-Zellen ein rekombinantes Plasmid, wie es in irgendeinem der Ansprüche 2 bis 8 beansprucht wird, inkorporiert wird, die transformierten Zellen kultiviert werden und das durch die genannten Zellen produzierte Thaumatin isoliert wird.

**Patentansprüche für den Vertragsstaat: AT**

1. Verfahren zur Herstellung von DNS-Sequenzen, die die verschiedenen allelen Formen von vollentwickeltem Thaumatin kodieren, dadurch gekennzeichnet, daß eine DNS-Sequenz, ausgewählt aus der aus verschiedenen allelen Formen des reifen Thaumatingens bestehenden Gruppe, angegeben in Fig. 2, nämlich:

32 Polynukleotiden, wovon eines die in Fig. 1 wiedergegebene Zusammensetzung hat und die anderen 31 die gleiche Zusammensetzung, angegeben in Fig. 1, aufweisen, jedoch mit der Ausnahme,

daß sie eine oder mehrere Variationen in den Nukleotiden 138 (entweder G oder C), 187 (entweder C oder A), 199 und 200 (entweder CG oder AA), 227 (entweder A oder G) und 337 (entweder G oder A) enthalten, synthetisch oder halb-synthetisch hergestellt wird, vorzugsweise unter Anwendung der Maßnahmen (1) bis (8), die in der Beschreibung zur partiellen Herstellung von Partialsequenzen des reifen Thaumatingens erwähnt werden.

2. Verfahren zur Herstellung rekombinanter Plasmide, dadurch gekennzeichnet, daß rekombinante Plasmide hergestellt werden, die DNS-Sequenzen, die irgendeine der verscheidenen allelen Formen des in Anspruch 1 beschriebenen reifen Thaumatins kodieren, und ein induzierbares oder konstitutives Regulon, das die Expression der genannten DNS-Sequenzen reguliert, enthalten.

3. Verfahren nach Anspruch 2, dadurch gekennzeichnet, daß ein Plasmid der pUR520-Familie, das ein Doppel-lac-UV5-System als Regulon enthält, hergestellt wird.

4. Verfahren nach Anspruch 2, dadurch gekennzeichnet, daß ein Plasmid der pUR530-Familie, das ein modifiziertes Tryptophan-System als Regulon enthält, hergestellt wird, wobei das modifizierte Tryptophan-System erhältlich ist durch Restriktionsendonuklease - *Hin*fl - Spaltung von trpED5, gefolgt von einer nachfolgenden Behandlung des DNS-Fragments von etwa 510 Basenpaaren, so erhalten durch Restriktionsendonuklease - *Taq*I in Gegenwart von *E. coli*-RNS-Polymerase, während die *Taq*I-Stelie in dem trp-REgulon geschützt ist, was zu einem Fragment führt, das 234 Basenpaare mit dem trp-Regulon enthält, darauf Behandeln des letzteren Fragmentes mit der S1-Nuklease, Ligation der glatten Enden mit dem *Eco*RI-Linker

$$(5')pGGAATTCC_{OH}(3'),$$

Schneiden mit *Eco*RI und nachfolgendes Klonieren in die *Eco*RI-Stelle vom pBR322.

5. Verfahren nach Anspruch 2, dadurch gekennzeichnet, daß ein Plasmid der pUR540-Familie, das das Polynukleotid 1128—1291 des M13-Gen-VIII-Systems als Regulon enthält, hergestellt wird.

6. Verfahren zur Herstellung von rekombinanten Plasmiden, beansprucht in Anspruch 2, wobei bei diesem Verfahren eine DNS-Sequenz, wie im Anspruch 1 beansprucht, mit der *Eco*RI-Stelle eines Regulons, wie im Anspruch 2 beschrieben, verbunden wird, dadurch gekennzeichnet, daß

(a) das 5'-Ende der genannten DNS-Sequenz mit einem Linker gekuppelt wird, der die Nukleotid-Sequenz

$$(5')pCAT(N)_nGAATTC(N')_nATG_{OH}(3')$$

aufweist, worin n=0, 1, 2 oder 3 ist und N und N' irgendwelche der Nukleotide A, T, G oder C sind, was unter der Bedingung gilt, daß in der Doppelstrangstruktur N und N' derartig sind, daß eine rotations-symmetrische Struktur vorliegt,

(b) das Kupplungsprodukt mit *Eco*RI behandelt wird und

(c) das *Eco*Ru-behandelte Kupplungsprodukt mit dem *Eco*RI-behandelten Regulon gekuppelt wird.

7. Verfahren zur Herstellung von reifem Thaumatin, dadurch gekennzeichnet, daß

(1) Plasmide, die entsprechend dem in irgendeinem der vorhergehenden Ansprüche 2 bis 6 beanspruchten Verfahren hergestellt wurden, den *E. coli*-Zellen inkorporiert werden,
(2) die transformierten Zellen kultiviert werden und
(3) das in diesen Zellen hergestellte Thaumatin isoliert wird.

8. Verfahren nach Anspruch 7, dadurch gekennzeichnet, daß ein nach dem Verfahren, wie es in den Ansprüchen 3, 4 und 5 beansprucht wird, hergestelltes Plasmid mit oder ohne AATT-Sequenz, die auf den in Anspruch 6 beschriebenen Linker zurückgeht, gelegen zwischen dem Regulon und dem reifen Thaumatingen des rekombinanten Plasmids, *E. coli*-Zellen inkorporiert wird.

**Revendications pour les Etats Contractants: BE, CH, DE, FR, GB, IT, LI, NL, SE**

1. Séquence d'ADN choisie dans la classe consistant en les diverses formes alléliques du gène de la thaumatine mature données à la Fig. 2, à savoir 32 polynucléotides dont l'un a la composition indiquée à la Fig. 1 et les 31 autres ont la même composition que celle indiquée à la Fig. 1 sauf qu'ils contiennent une ou plusieurs variantes dans les nucléotides 138 (G ou C), 187 (C ou A), 199 et 200 (CG ou AA), 227 (A ou G) et 337 (G ou A).

2. Plasmides recombinants comprenant

(i) une séquence d'ADN suivant la revendication 1 et
(ii) un régulon inductible ou constitutif qui régule l'expression de ces séquences d'ADN.

3. Plasmides recombinants suivant la revendication 2, comprenant un régulon inductible consistant en un système double lac UV5 régulant l'expression des gènes structuraux.

4. Plasmide recombinant suivant la revendication 3, qui peut être obtenu par préparation à partir

(a) du plasmide pUR201,

(b) l'une des 32 séquences d'ADN codant pour la thaumatine de la Fig. 2, et

(c) le groupe de liaison d'*Eco*RI

$$(5')pCAT(N)_nGAATTC(N')_nATG_{OH}(3'),$$

où n=0, 1, 2 ou 3 et N et N' sont l'un quelconque des nucléotides A, T, G ou C, avec la restriction que dans la structure en double hélice, N et N' sont tels qu'une structure symétrique de rotation soit présente,

étant entendu que le plasmide pUR201 peut être obtenu par

(a.1) coupure du plasmide pBR322 par *Eco*RI,

(a.2) coupure du plasmide pKB268 par *Eco*RI et isolement du fragment d'ADN codant pour le régulon double lac (UV5),

(a.3) liaison du fragment d'ADN contenant le régulon et du pBR322 coupé par *Eco*RI,

(a.4) coupure sélective du plasmide résultant par *Eco*RI, en présence d'ARN polymérase, suivie d'un traitement par la nucléase S1, puis d'une liaison par la ligase T4, conduisant à un plasmide (pUR201) dans lequel le site *Eco*RI le plus distant du site *Hin*dIII dans le gène de résistance de la tétracycline est sélectivement éliminé.

5. Plasmides recombinants suivant la revendication 2, comprenant un système tryptophane modifié régulant l'expression des gènes structuraux, lequel système tryptophane modifié peut être obtenu par coupure de trp ED5 par

l'endonucléase de restriction *Hin*fI, suivie d'un traitement ultérieur du fragment d'ADN d'environ 510 paires ds bases ainsi obtenu au moyen de l'endonucléase de restriction *Taq*I en présence d'ARN polymérase d'*E. coli*, tout en protégeant le site *Taq*I dans le régulon trp, conduisant à un fragment contenant 234 paires de bases comprenant le régulon trp, puis par traitement de ce dernier fragment au moyen de la nucléase S1, liaison de la coupure franche avec le groupe de liaison d'*Eco*RI

$$(5')pGGAATTCC_{OH}(3'),$$

coupure au moyen d'*Eco*RI et ensuite clonage dans le site *Eco*RI de pBR322.

6. Plasmide recombinant suivant la revendication 5, qui peut être obtenu par préparation à partir (a) du plasmide pUR301

(b) de l'une des 32 séquences d'ARN codant pour la thaumatine de la Fig. 2, et

(c) du groupe de liaison d'*Eco*RI

$$(5')pCAT(N)_nGAATTC(N')_nATG_{OH}(3'),$$

où n=0, 1, 2 ou 3 et N et N' sont l'un quelconque des nucléotides A, T, G ou C, avec la restriction que dans la structure en double hélice N et N' sont tels qu'une structure symétrique de rotation soit présente,

étant entendu que le plasmide pUR301 peut être obtenu par coupure sélective du plasmide issu de pBR322 contenant le régulon tryptophane modifié décrit à la revendication 5 par *Eco*RI, en présence de bromure d'éthidium, suivie d'un traitement par la nucléase S1 et ensuite de la liaison par la ligase T4, conduisant à un plasmide (pUR301) dans lequel le site *Eco*RI le plus distant du site *Hin*dIII dans le gène de résistance à la tétracycline est sélectivement éliminé.

7. Plasmides recombinants suivant la revendication 2, comprenant un système modifié du gène VIII de M13 régulant l'expression des gènes structuraux, lequel système modifié du gène VIII de M13 comprend de l'ADN en double hélice consistant en les nucléotides 1128—1291.

8. Plasmide recombinant suivant la revendication 7, qui peut être obtenu par préparation à partir

(a) du plasmide pUR401,

(b) de l'une des 32 séquences d'ADN codant la thaumatine de la Fig. 2, et

(c) du groupe de liaison d'*Eco*RI

$$(5')pCAT(N)_nGAATTC(N')_nATG_{OH}(3'),$$

où n=0, 1, 2 ou 3 et N et N' sont l'un quelconque des nucléotides A, T, G et C, avec la restriction que dans la structure en double hélice, N et N' sont tels qu'une structure symétrique de rotation soit présente,

étant entendu que le plasmide pUR401 est obtenu par

(a.1) liaison, par la coupure franche, du groupe de liaison d'*Eco*RI

$$(5')pGGAATTCC_{OH}(3')$$

avec le région modifié du gène VIII de M13 consistant en les polynucléotides 1128—1293 (qui peut s'obtenir par traitement ultérieur d'ADN de RFM13 avec *Taq*I et *Hae*III, traitement de réparation du site *Taq*I avec de l'ADN polymérase d'*E. coli*, digestion partielle du fragment avec *Mnl*I, puis traitements successifs avec l'ADN polymérase T4 et la nucléase S1),

(a.2) traitement du fragment résultant de (a.1) avec *Eco*RI, suivi de liaison avec pBR322 coupé par *Eco*RI,

(a.3) coupure sélective du plasmide issu de pBR322 contenant le régulon modifié du gène VIII de M13 par *Eco*RI en présence de bromure d'éthidium, suivie du traitement par la nucléase S1 et ensuite de la liaison avec la ligase T4, conduisant à un plasmide (pUR401) dans lequel le site *Eco*RI le plus distant du site *Hin*dIII dans le gène de résistance à la tétracycline est sélectivement éliminé.

9. Procédé de préparation de plasmides recombinants suivant la revendication 2, dans lequel procédé une séquence d'ADN suivant la revendication 1 est liée au site *Eco*RI d'un régulon tel que décrit dans la revendication 1, caractérisé en ce que

(a) l'extrémité 5' de cette séquence d'ADN est couplée avec un groupe de liaison comportant la séquence de nucléotides

$$(5')pCAT(N)_nGAATTC(N')_nATG_{OH}(3')$$

où n=0, 1, 2 ou 3 et N et N' sont l'un quelconque des nucléotides A, T, G ou C, avec la restriction que dans la structure en double hélice, N et N' sont tels qu'une structure symétrique de rotation soit présente,

(b) le produit de couplage est traité par *Eco*RI et

(c) le produit de couplage traité par *Eco*RI est couplé avec le régulon traité par *Eco*RI.

10. Culture bactérienne comprenant des cellules d'*E. coli* contenant un ou plusieurs des plasmides recombinants suivant les revendications 2—8 avec ou sans la séquence AATT provenant du groupe de liaison comme décrit dans la revendication 9 située entre le régulon et le gène structural du plasmide recombinant.

11. Culture bactérienne suivant la revendication 10, dans laquelle le plasmide recombinant est choisi dans la classe consistant en les plasmides suivant les revendications 4, 6 et 8.

12. Procédé de production de la thaumatine mature, qui comprend l'incorporation d'un plasmide recombinant suivant l'une quelconque des revendications 2—8 dans des cellules d'*E. coli*, la culture des cellules transformées et l'isolement de la thaumatine produite par ces cellules.

**Revendications pour l'Etat Contractant: AT**

1. Procédé de production de séquences d'ADN codant pour les différentes formes alléliques de la

thaumatine mature, caractérisé en ce qu'une séquence d'ADN sélectionnée dans la classe consistant en les diverses grandes formes alléliques du gène de la thaumatine mature donnée à la Fig. 2 à savoir:

32 polynucléotides dont l'un a la composition indiquée à la Fig. 1 et les 31 autres ont la même composition que celle indiquée à la Fig. 1 sauf qu'ils contiennent une ou plusieurs variantes dans les nucléotides 138 (G ou C), 187 (C ou A), 199 et 200 (CG ou AA), 227 (A ou G) et 337 (G ou A) est préparée par voie synthétique ou semi-synthétique, de préférence suivant les stades (1)—(8) mentionnés dans le mémoire pour la préparation partielle de séquences partielles du gène de la thaumatine mature.

2. Procédé de production de plasmides recombinants, caractérisé en ce qu'on produit des plasmides recombinants qui comprennent des séquences d'ADN codant pour l'une quelconque des diverses formes alléliques de la thaumatine mature comme décrit dans la revendication 1 et un régulon inductible ou constitutif qui régule l'expression de ces séquences d'ADN.

3. Procédé suivant la revendication 2, caractérisé en ce qu'on produit un plasmide de la famille pUR520 contenant un système double lac UV5 comme régulon.

4. Procédé suivant la revendication 2, caractérisé en ce qu'on produit un plasmide de la famille pUR530 contenant un système tryptophane modifié comme régulon, lequel système tryptophane modifié peut être obtenu par coupure de trpED5 par l'endonucléase de restriction *Hin*fI, suivie d'un traitement ultérieur du fragment d'ADN d'environ 510 paires de bases ainsi obtenu au moyen de l'endonucléase de restriction *Taq*I en présence d'ARN polymérase d'*E. coli*, tout en protégeant le site *Taq*I dans le régulon trp, conduisant à une fragment contenant 234 paires de bases comprenant le régulon trp, puis par traitement de ce dernier fragment au moyen de la nucléase S1, liaison de la coupure franche avec le groupe de liaison d'*Eco*RI

$$(5')pGGAATTCC_{OH}(3'),$$

coupure au moyen d'*Eco*RI et ensuite clonage dans le site *Eco*RI de pBR322.

5. Procédé suivant la revendication 2, caractérisé en ce qu'on produit un plasmide de la famille pUR540 contenant le polynucléotide 1128—1291 du système du gène VIII de M13 comme régulon.

6. Procédé de préparation de plasmides recombinants suivant la revendication 2, dans lequel procédé une séquence d'ADN suivant la revendication 1 est liée au site *Eco*RI d'un régulon tel que décrit dans la revendication 2, caractérisé en ce que

(a) l'extrémité 5' de cette séquence d'ADN est couplée avec un groupe de liaison comportant la séquence de nucléotides

$$(5')pCAT(N)_nGAATTC(N')_nATG_{OH}(3')$$

où n=0, 1, 2 ou 3 et N et N' sont l'un quelconque des nucléotides A, T, G ou C, avec la restriction que dans la structure en double hélice, N et N' sont tels qu'une structure symétrique de rotation soit présente,

(b) le produit de couplage est traité par *Eco*RI et

(c) le produit de couplage traité par *Eco*RI est couplé avec le régulon traité par *Eco*RI.

7. Procédé de production de la thaumatine mature, caractérisé en ce que

(1) des plasmides produits par un procédé suivant l'une quelconque des revendications 2—6 sont incorporés à des cellules d'*E. coli*;
(2) les cellules transformées sont cultivées, et
(3) la thaumatine produite par ces cellules est isolée.

8. Procédé suivant la revendication 7, caractérisé en ce qu'un plasmide produit par une procédé suivant les revendications 3, 4 et 5, avec ou sans la séquence AATT provenant du groupe de liaison comme décrit dans la revendication 6 située entre le régulon et le gène de thaumatine mature du plasmide recombinant, est incorporé à des cellules d'*E. coli*.

Fig.1.

Amino acid sequence and DNA sequence of (mature) thaumatin

```
1
ALA THR PHE GLU ILE VAL ASN ARG CYS SER TYR THR VAL TRP ALA ALA ALA SER LYS GLY
GCC ACC TTC GAG ATC GTC AAC CGC TGC TCC TAC ACC GTG TGG GCG GCC GCC TCC AAA GGC

61
ASP ALA ALA LEU ASP ALA GLY GLY ARG GLN LEU ASN SER GLY GLU SER TRP THR ILE ASN
GAC GCC GCC CTG GAC GCC GGC GGC CGC CAG CTC AAC TCG GGA GAG TCC TGG ACC ATC AAC

121
VAL GLU PRO GLY THR LYS GLY GLY LYS ILE TRP ALA ARG THR ASP CYS TYR PHE ASP ASP
GTA GAA CCC GGC ACC AAG GGT GGC AAA ATC TGG GCC CGC ACC GAC TGC TAT TTC GAC GAC

181
SER GLY ARG GLY ILE CYS ARG THR GLY ASP CYS GLY GLY LEU LEU GLN CYS LYS ARG PHE
AGC GGC CGC GGC ATC TGC CGG ACC GGC GAC TGC GGC GGC CTC CTC CAG TGC AAG CGC TTC

241
GLY ARG PRO PRO THR THR LEU ALA GLU PHE SER LEU ASN GLN TYR GLY LYS ASP TYR ILE
GGC CGG CCG CCC ACC ACG CTG GCG GAG TTC TCG CTC AAC CAG TAC GGC AAG GAC TAC ATC

301
ASP ILE SER ASN ILE LYS GLY PHE ASN VAL PRO MET ASP PHE SER PRO THR THR ARG GLY
GAC ATC TCC AAC ATC AAA GGC TTC AAC GTG CCG ATG GAC TTC AGC CCG ACC ACG CCC GGC

361
CYS ARG GLY VAL ARG CYS ALA ALA ASP ILE VAL GLY GLN CYS PRO ALA LYS LEU LYS ALA
TGC CGC GGG GTG CGG TGC GCC GCC GAC ATC GTG GGG CAG TGC CCG GCG AAG CTG AAG GCG

421
PRO GLY GLY GLY CYS ASN ASP ALA CYS THR VAL PHE GLN THR SER GLU TYR CYS CYS THR
CCG GGG GGT GGT TGC AAC GAT GCG TGC ACC GTG TTC CAG ACG AGC GAG TAC TGC TGC ACC

481
THR GLY LYS CYS GLY PRO THR GLU TYR SER ARG PHE PHE LYS ARG LEU CYS PRO ASP ALA
ACG GGG AAG TGC GGG CCG ACG GAG TAC TCG CGC TTC TTC AAG AGG CTT TGC CCG GAC GCG

541
PHE SER TYR VAL LEU ASP LYS PRO THR THR VAL THR CYS PRO GLY SER SER ASN TYR ARG
TTC AGT TAT GTC CTG GAC AAG CCA ACC ACC GTC ACC TGC CCC GGC AGC TCC AAC TAC AGG

601
VAL THR PHE CYS PRO THR ALA
GTC ACT TTC TGC CCT ACT GCC
```

Fig.2.

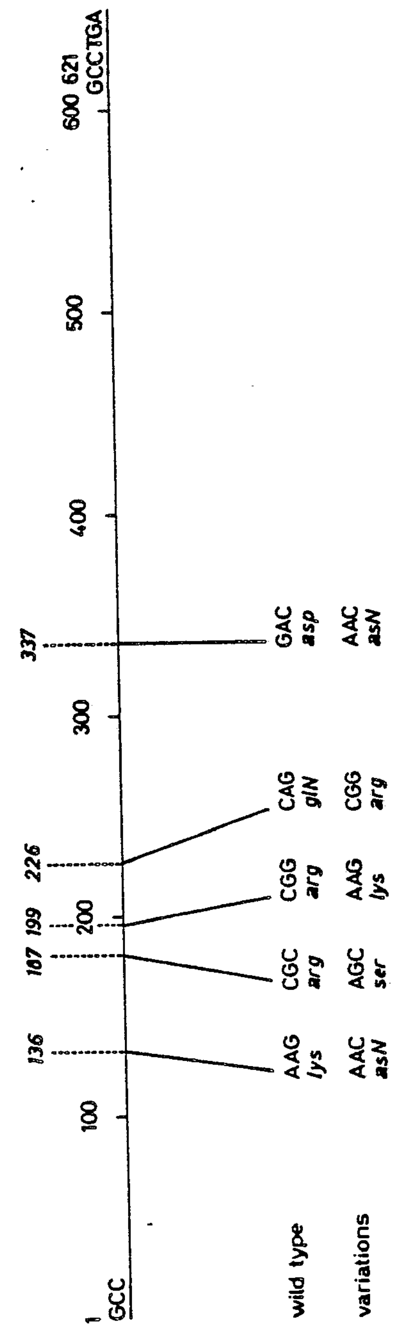


Fig. 2 Allelic variations in the thaumatin gene.

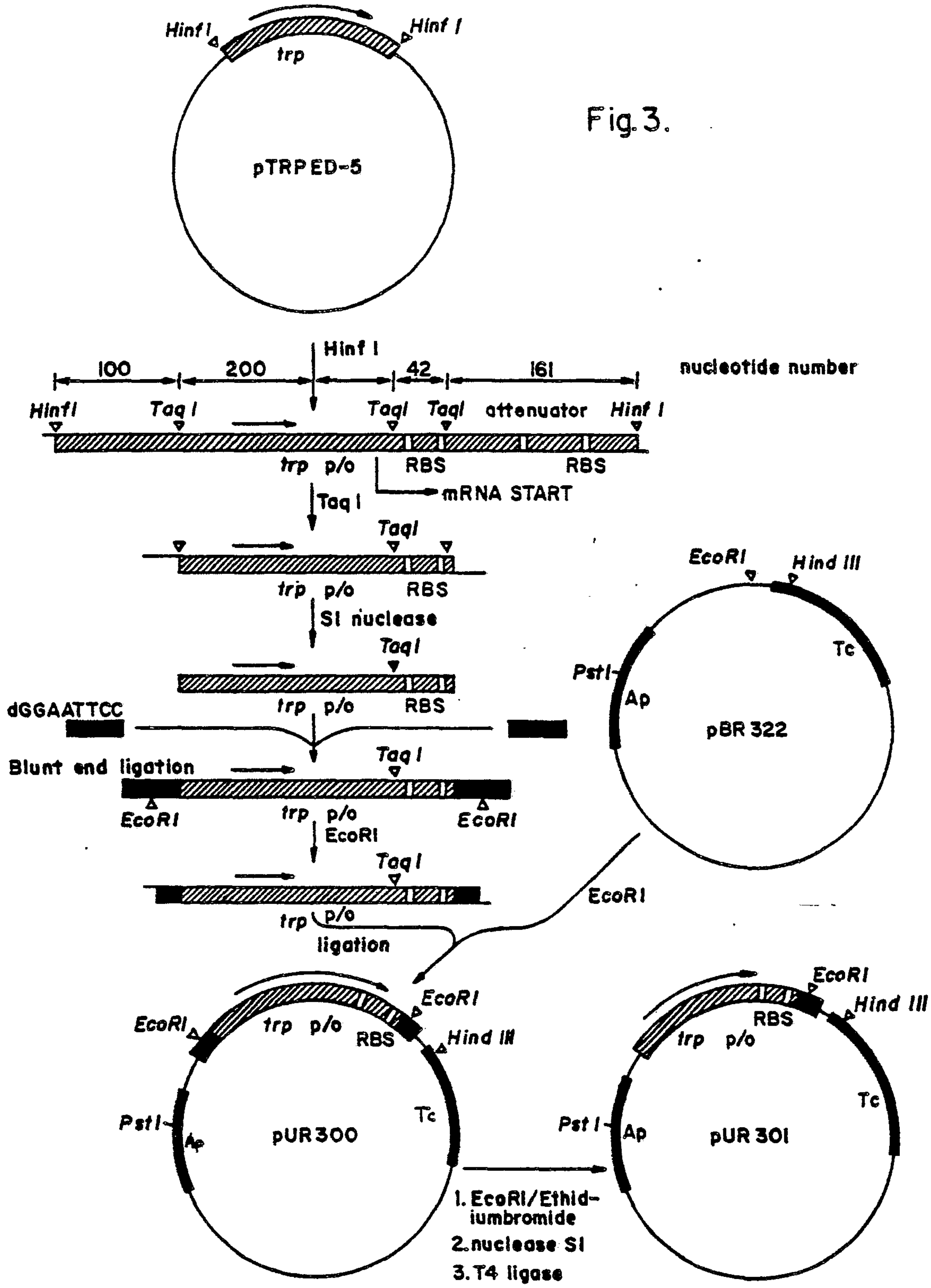

Hinf I
Hinf I
trp
pTRPED-5
Hinf I
100
200
42
161
nucleotide number
Hinf I
Taq I
Taq I
Taq I
attenuator
Hinf I
trp p/o
RBS
RBS
mRNA START
Taq I
Taq I
trp p/o
RBS
S1 nuclease
Taq I
trp p/o
RBS
dGGAATTCC
Blunt end ligation
Taq I
EcoRI
trp p/o
EcoRI
EcoRI
Taq I
trp p/o
ligation
EcoRI
Hind III
Tc
PstI
Ap
pBR 322
EcoRI
EcoRI
EcoRI
trp p/o
RBS
Hind III
PstI
Ap
pUR300
Tc
1. EcoRI/Ethidiumbromide
2. nuclease S1
3. T4 ligase
EcoRI
Hind III
RBS
trp p/o
Tc
PstI
Ap
pUR301


Fig. 3.

Fig. 4.

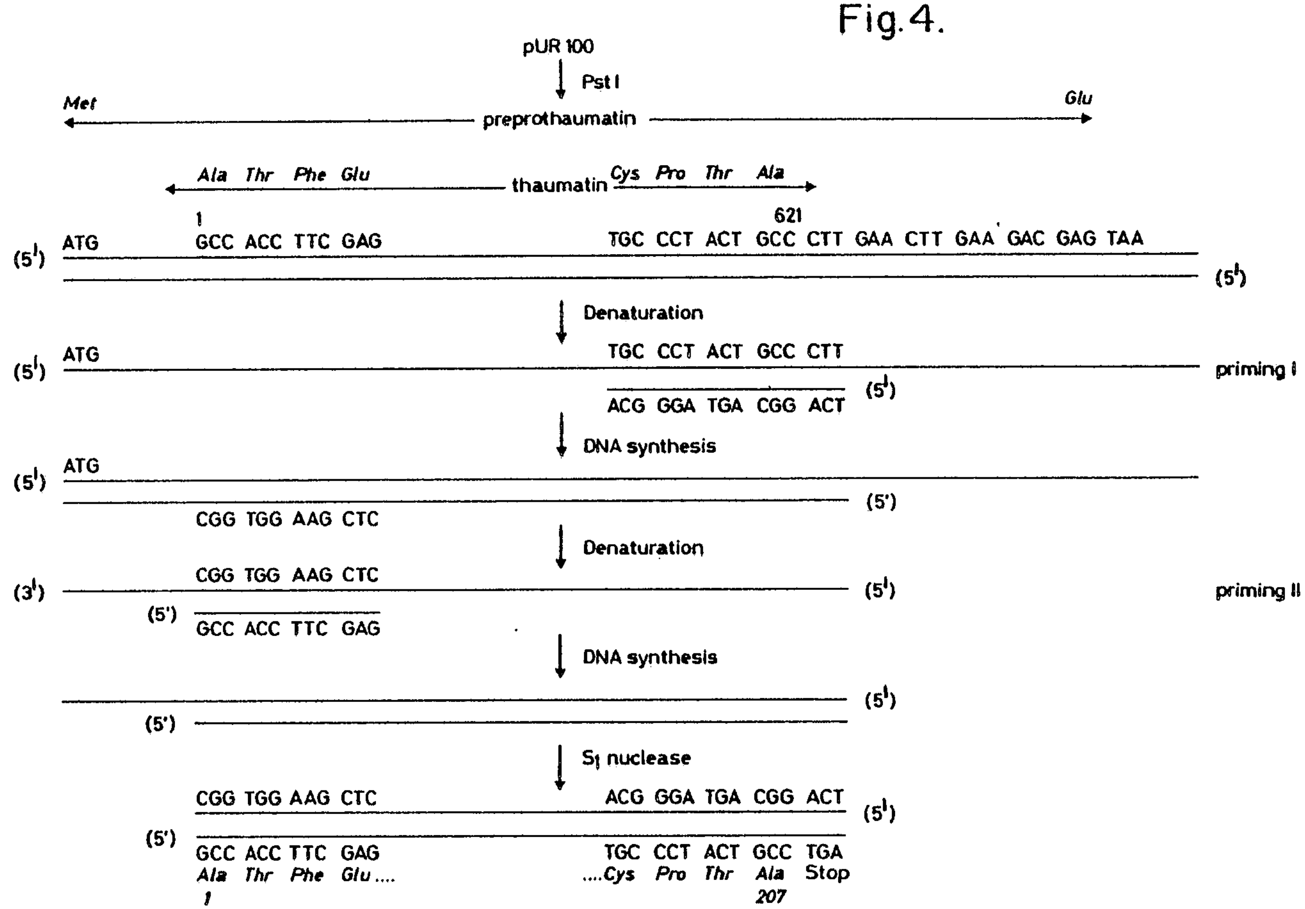


Fig. 4 Construction of the DNA sequence encoding the mature fully processed thaumatin.

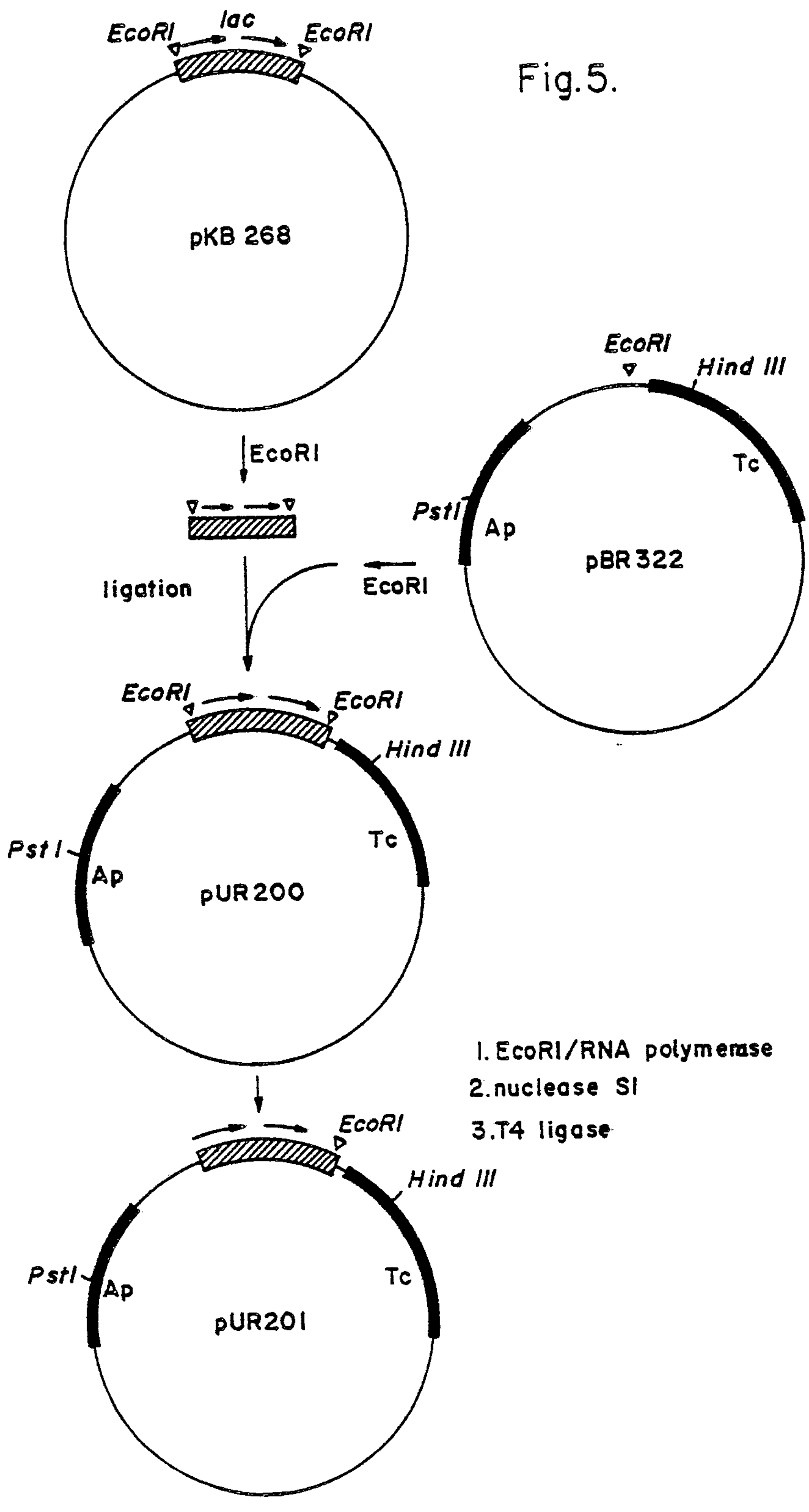
EcoRI
lac
EcoRI
pKB 268
EcoRI
ligation
EcoRI
EcoRI
Hind III
PstI
Ap
Tc
pBR 322
EcoRI
EcoRI
Hind III
Pst I
Ap
Tc
pUR 200
1. EcoRI/RNA polymerase
2. nuclease SI
3. T4 ligase
EcoRI
Hind III
PstI
Ap
Tc
pUR201

Fig. 5.

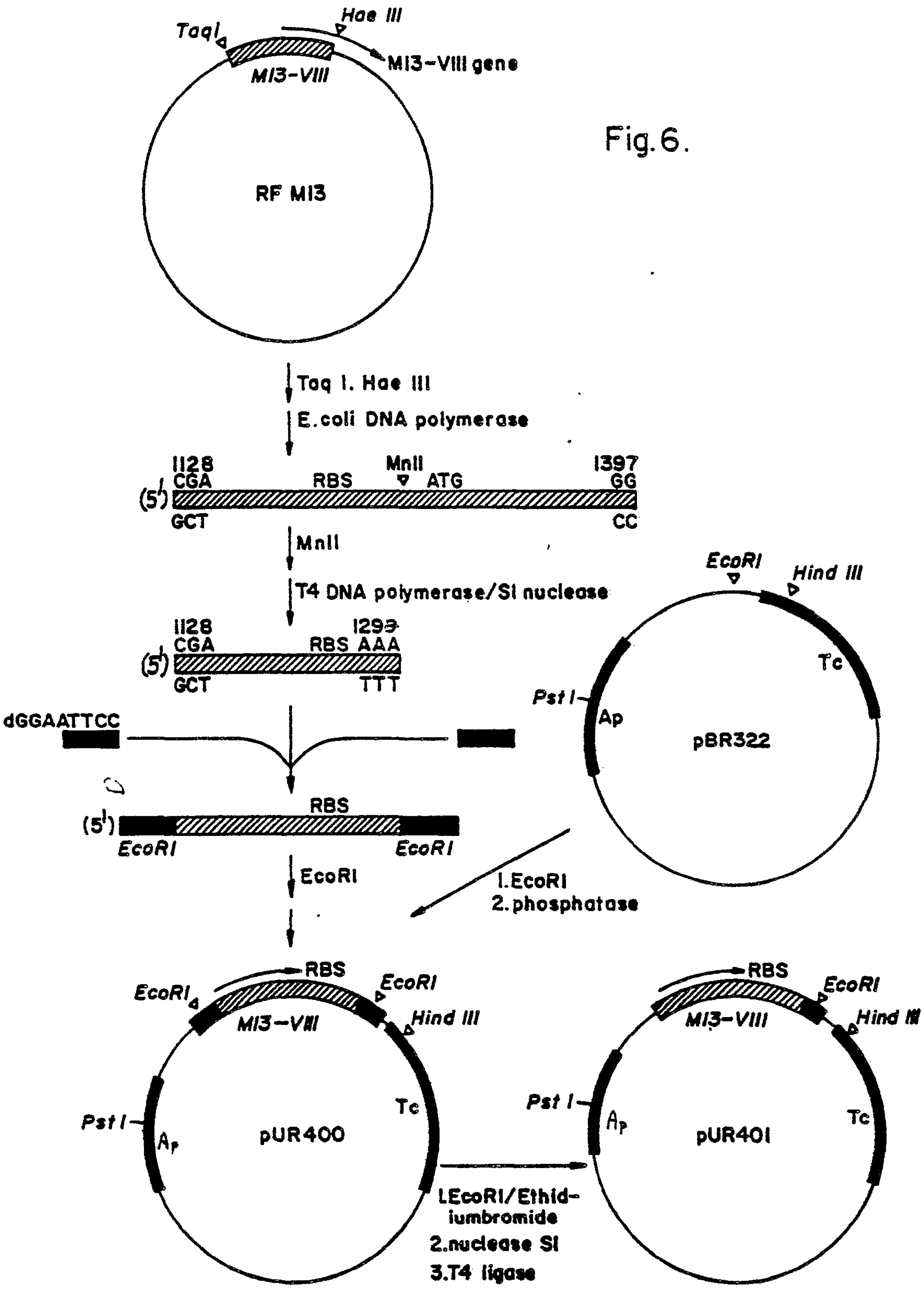
TaqI
Hae III
M13-VIII
M13-VIII gene
RF M13
Taq I. Hae III
E.coli DNA polymerase
1128
CGA
RBS
MnII
ATG
1397
GG
(5')
GCT
CC
MnII
T4 DNA polymerase/SI nuclease
1128
CGA
RBS
1293
AAA
(5')
GCT
TTT
dGGAATTCC
RBS
(5')
EcoRI
EcoRI
EcoRI
EcoRI
Hind III
PstI
Ap
Tc
pBR322
1.EcoRI
2.phosphatase
RBS
EcoRI
EcoRI
M13-VIII
Hind III
PstI
Ap
Tc
pUR400
1.EcoRI/Ethidiumbromide
2.nuclease SI
3.T4 ligase
RBS
EcoRI
M13-VIII
Hind III
PstI
Ap
Tc
pUR401

Fig. 6.

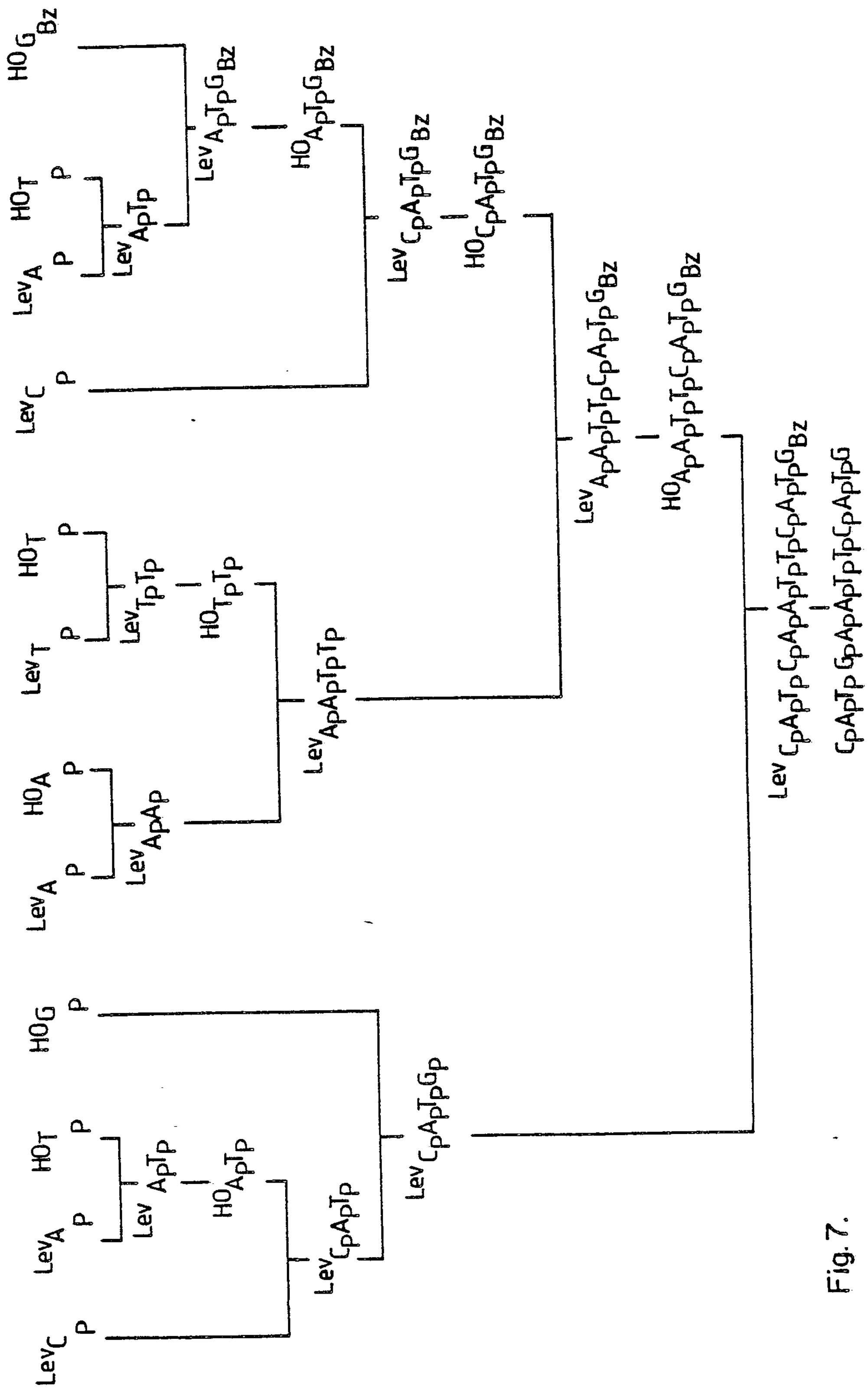
HOGBz
LevA p
HOT p
LevApTp
LevApTpGBz
HOApTpGBz
LevC p
LevCpApTpGBz
HOCpApTpGBz
LevT p
HOT p
LevTpTp
HOTpTp
LevA p
HOA p
LevApAp
LevApApTpTp
LevApApTpTpCpApTpGBz
HOApApTpTpCpApTpGBz
LevC p
LevA p
HOT p
LevApTp
HOApTp
LevCpApTp
HOG p
LevCpApTpGp
LevCpApTpCpApApTpTpCpApTpGBz
CpApTpGpApApTpTpCpApTpG

Fig. 7.

# Fig.8.

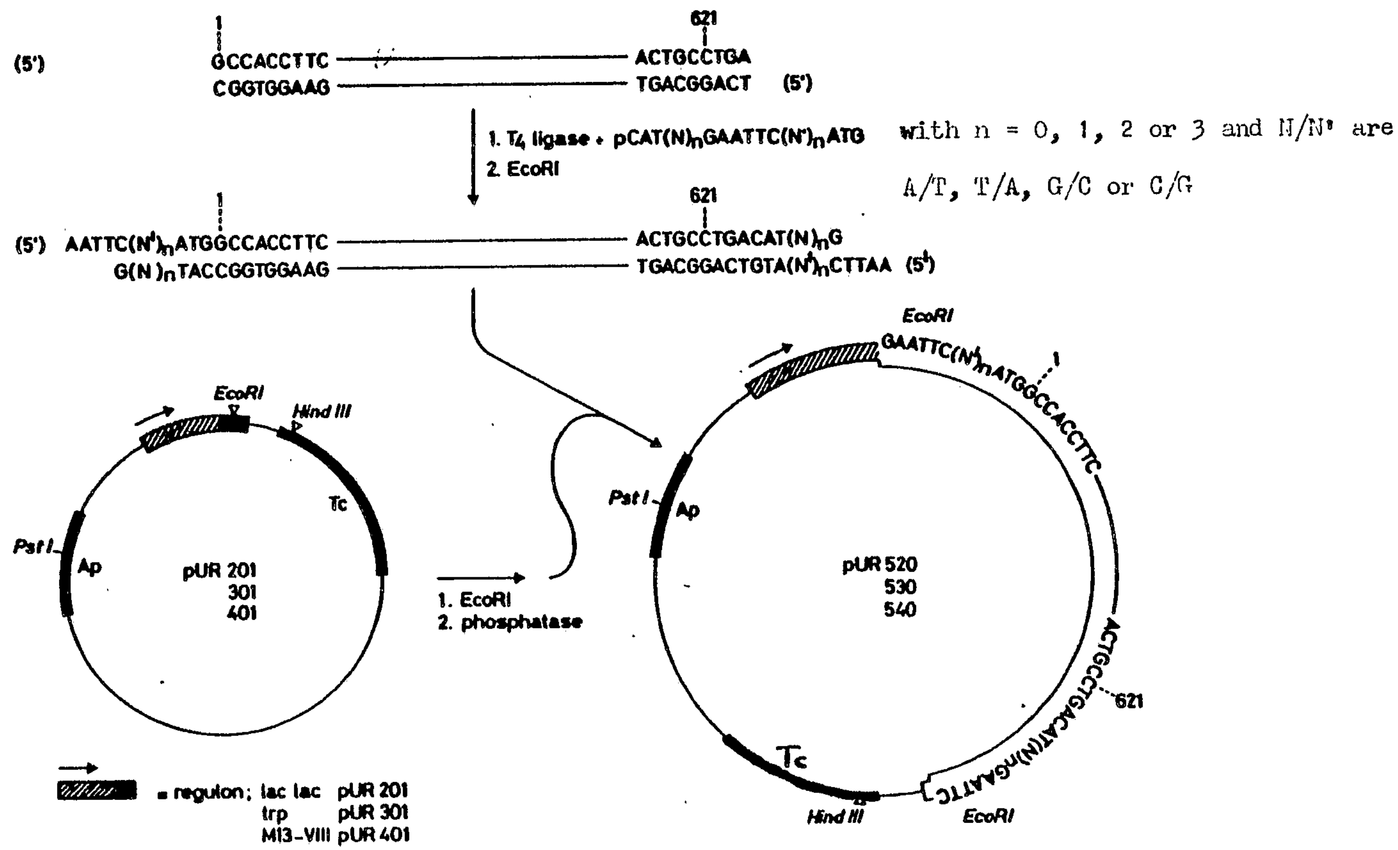


Fig. 8 Construction of pUR 520, pUR 530 and pUR 540.